(19) Europäisches Patentamt
European Patent Office
Office européen des brevets

(11) Publication number: **0 506 011 A1**

# (12) EUROPEAN PATENT APPLICATION

(21) Application number: **92105134.8**

(22) Date of filing: **25.03.92**

(51) Int. Cl.5: **C07D 405/04**, C07D 409/04, A61K 31/41

(30) Priority: **26.03.91 JP 84357/91**

(43) Date of publication of application: **30.09.92 Bulletin 92/40**

(84) Designated Contracting States: **DE FR GB IT**

(71) Applicant: **Wakamoto Pharmaceutical Co., Ltd.**
**5-3, Nihonbashimuro-machi 1-chome**
**Chuo-ku, Tokyo(JP)**

(72) Inventor: **Horio, Yoshihiro**
**2203-4, Minami-Yana**
**Hatano-shi, Kanagawa-ken(JP)**
Inventor: **Ootake, Yasuhiro**
**2266-2, Tsukahara**
**Minamiashigara-shi, Kanagawa-ken(JP)**
Inventor: **Sawaki, Shohei**
**1479-22, Ninomiya, Ninomiya-machi**
**Naka-gun, Kanagawa-ken(JP)**
Inventor: **Inukai, Sinji**
**2-2-1-402, Minamigaoka**
**Hatano-shi, Kanagawa-ken(JP)**
Inventor: **Agata, Mitsuzi**
**149-1, Kanate, Oi-machi**
**Ashigarakami-gun, Kanagawa-ken(JP)**
Inventor: **Umezawa, Manami**
**1-15-2, Morinosato**
**Atsugi-shi, Kanagawa-ken(JP)**
Inventor: **Goto, Masayoshi**
**4-2-5-408, Higashi-Naruse**
**Isehara-shi, Kanagawa-ken(JP)**

(74) Representative: **Vossius & Partner**
**Siebertstrasse 4 P.O. Box 86 07 67**
**W-8000 München 86(DE)**

## (54) Tetrazoleacetic acid derivatives having aldose reductase inhibitory activity.

(57) A tetrazoleacetic acid derivative represented by the following general Formula (I):

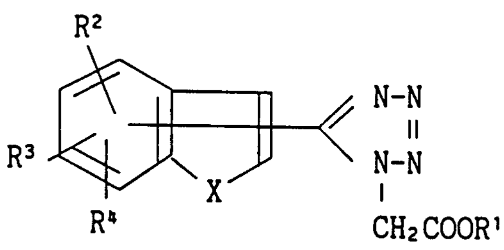

(I)

[in Formula (I), $R^1$ represents a hydrogen atom or a lower alkyl group; $R^2$, $R^3$ and $R^4$ are the same or different from each other and are selected from the group consisting of a hydrogen atom, a lower alkyl group, a halogen atom and a lower alkoxy group; X is an oxygen atom or a sulfur atom; and the tetrazole group may substituent may be present arbitrarily at the 2-,3-,4-,5-,6- and 7-position of the benzofurane or benzothiophene ring] or a salt thereof shows excellent aldose reductase inhibitory activity, has low toxicity to organisms and is quite effective as an essential component of a preventive medicine and/or remedy for diabetic complications.

EP 0 506 011 A1

The present invention relates to a compound having an aldose reductase inhibitory activity and more specifically to a tetrazoleacetic acid derivative and an aldose reductase inhibitor which comprises the tetrazoleacetic acid derivative as an effective component and which is effective as a preventive medicine and/or remedy for diabetic complications as well as to its use for preparing pharmaceuticals for alleviating or reducing diabetic complications.

It has been known that aldose reductase inhibitors are effective for prevention and/or treatment of diabetic complications. This is detailed in the article of Dr. Tsuyoshi TANIMOTO [Division of Biological Chemistry and Reference Standards, National Institute of Hygienic Sciences] (see Farumashia, 1988, 24, No.5, pp. 459-463).

This article discloses the chemical structures and 50% inhibitory concentrations ($IC_{50}$) of representative aldose reductase inhibitors such as Alrestatin, Tolrestat, 4-Isopropyl-BPOC, Sorbinil, M-79175, Alconil, ADN-138, Epalrestat, CT-112 and Statil.

The inventors of this invention conducted screening of novel aldose reductase inhibitors and found that tetrazoleacetic acid derivatives have very high aldose reductase inhibitory activity. They filed two US patent applications (USSN 07/497,500 and USSN 07/588,057).

An object of the present invention is generally to provide a compound which shows excellent aldose reductase inhibitory activity, has low toxicity to organisms and is quite effective as a preventive medicine and/or remedy for diabetic complications and more specifically to provide a tetrazoleacetic acid derivative.

Another object of the present invention is to provide an aldose reductase inhibitor which comprises a tetrazoleacetic acid derivative as an effective component and which is effective as a preventive medicine and/or remedy for diabetic complications.

A further object of the present invention is to provide a method for alleviating or reducing symptoms related to diabetic complications. According to an aspect of the present invention, there is provided a novel tetrazoleacetic acid derivative represented by the following general Formula (I).

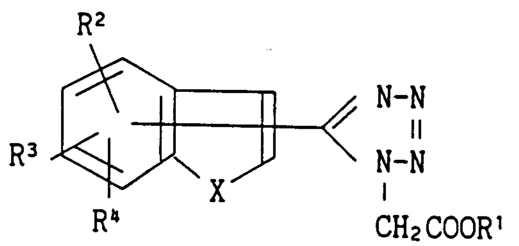

(I)

[in Formula (I), R' represents a hydrogen atom or a lower alkyl group; $R^2$, $R^3$ and $R^4$ are the same or different from each other and are selected from the group consisting of a hydrogen atom, a lower alkyl group, a halogen atom and a lower alkoxy group; X is an oxygen atom or a sulfur atom; and the tetrazole group substituent may be located arbitrarily at the 2-,3-,4-,5-, 6- and 7-position of the benzofurane or benzothiophene ring] or a salt thereof.

According to another aspect of the present invention, there is provided an aldose reductase inhibitor which comprises a tetrazoleacetic acid derivative represented by the Formula (I) [in Formula (I), $R^1$, $R^2$, $R^3$, $R^4$ and X is the same meaning as the above and the tetrazole group substituent may be located arbitrarily at 2-, 3-,4-,5-,6- and 7-position of the benzofurane or benzothiophene ring] or a salt thereof, and a pharmaceutically acceptable carrier.

The tetrazoleacetic acid derivatives and the aldose reductase inhibitor as well as the method for alleviating diabetic complications according to the present invention will hereunder be explained in more detail.

First, each substituent in Formula (I) will be explained in detail.

The lower alkyl group represented by $R^1$ is, for instance, methyl, ethyl, propyl, isopropyl, butyl, isobutyl or t-butyl group; the lower alkyl group by $R^2$, $R^3$ or $R^4$ is, for instance, methyl, ethyl, propyl, isopropyl, butyl, isobutyl and t-butyl groups; examples of the lower alkoxy group by $R^2$, $R^3$ or $R^4$ are methoxy, ethoxy, propoxy, isopropoxy, butoxy, isobutoxy and t-butoxy groups; the halogen atom by $R^2$, $R^3$ or $R^4$ is, for instance, fluorine, chlorine or bromine. The substituents of$R^2$, $R^3$ and $R^4$ and the tetrazole group may be present on any position on the benzofurane ring or benzothiophene ring.

In addition, salts of the foregoing compounds represented by Formula (I) wherein $R^1$ is a hydrogen atom are pharmaceutically acceptable ones and typical examples thereof include inorganic salts such as alkali metal salts (for instance, sodium salts and potassium salts), alkaline earth metal salts (for instance,

calcium salts and magnesium salts) and ammonium salts; and organic salts such as organic amine salts (for instance, triethylamine salts, pyridine salts and ethanolamine salts) and salts with basic amino acids, for instance, arginine.

The aldose reductase inhibitors according to the present invention comprises, as an essential component, at least one compound represented by the foregoing general formula (I) and are effective as preventive medicines and/or remedies for diabetic complications. It has been known that the term "diabetic complications" means a variety of pathema such as peripheral disorder, retinopathy, nephrosis, cataract and keratopathy. These diseases or disorders are triggered by hyperglycemia resulted from the diabetic disease, that the production of sorbitol in the polyol metabolic pathway is correspondingly abnormally accelerated and that, as a result, a large amount of sorbitol is accumulated within cells. This leads to the onset of these diseases.

The aldose reductase inhibitors of the present invention can suppress the sorbitol-production through strong inhibition of the activity of the aldose reductase which catalyzes the sorbitol-production in the foregoing polyol metabolic pathway and thus show excellent preventive and/or treating effects for these various diabetic complications.

The dose of the compounds of Formula (I) is appropriately determined depending on the conditions or symptoms of patients to be treated; but in general ranges from 1 to 1,000 mg per day for an adult which is administered at one time or over several times. The compounds may be administered through any route for medication such as oral or parenteral administration, subcutaneous injection, intravenous injection and local administration.

The aldose reductase inhibitors of the present invention may usually comprise, in addition to the foregoing compounds as the essential components, pharmaceutically acceptable carriers, vehicles and other additives. The inhibitors of the invention may be used in any dosage form such as tablets, powders, fine particles, granules, capsules, pills, liquid preparations, solutions and suspensions for injection and eye drops.

Methods for preparing the compounds (I) as the essential components, conditions therefor or the like will be explained in more detail below with reference to the following reaction schemes.

Reaction Scheme 1: Synthesis of tetrazole ring

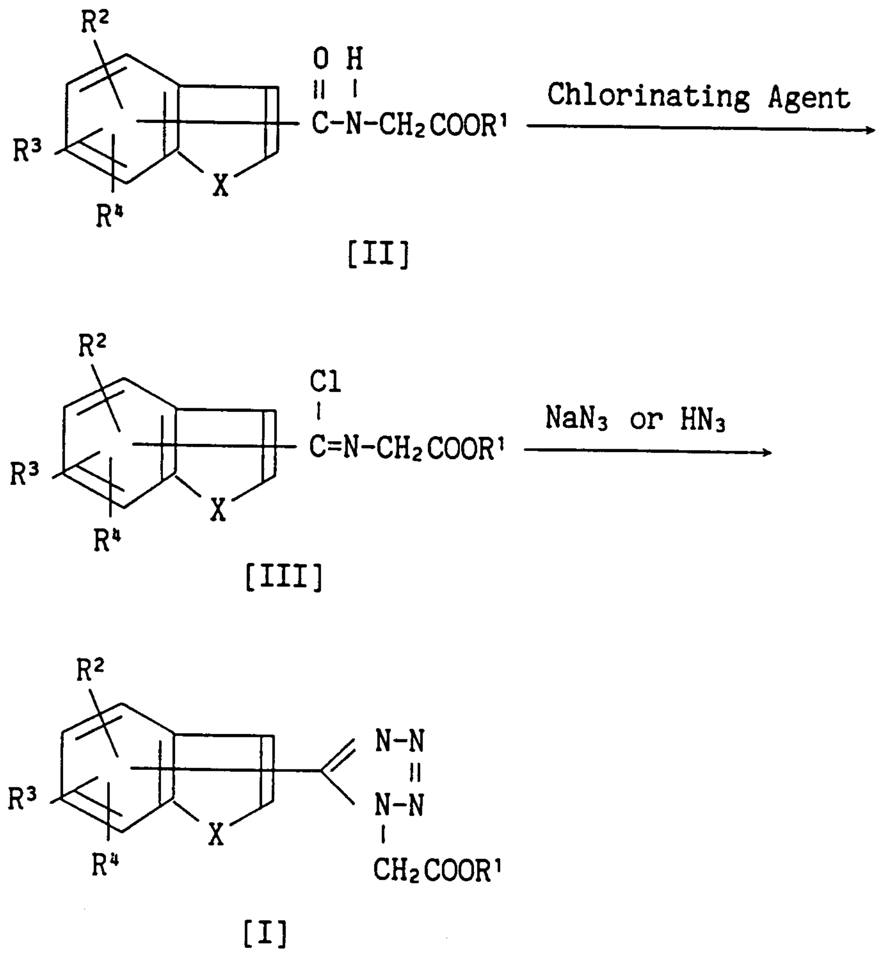


(wherein $R^1$, $R^2$, $R^3$, $R^4$ and X have the same meaning as described hereinbefore)

Reaction Scheme 2: Conversion of $R^1$ to hydrogen atom

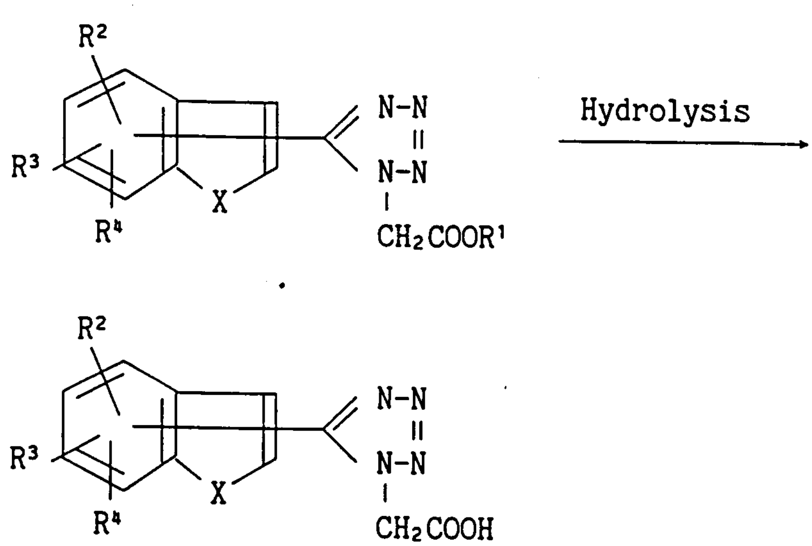


(wherein $R^1$, $R^2$, $R^3$, $R^4$ and X have the same meaning as described hereinbefore)

The reaction scheme 1 presents the reaction for production of the tetrazole ring. The reaction can be

performed according to the production method described in the Journal of Medicinal Chemistry, vol.13, 725, (1970) or the Synthesis, 725 (1973). In this reaction, the carbamide acetate of Formula (II) is reacted with a chlorinating agent such as phosphorus pentachloride, thionyl chloride or thionyl chloride-N,N-dimethylformamide to produce a corresponding imidoyl chloride (III), which is then reacted with sodium azide or hydrogen azide to give the object compound of Formula (I). The reaction for obtaining the imidoyl chloride can be carried out in an organic solvent such as benzene, toluene or methylene chloride. In general, the reaction is preferably performed at room temperature. In the subsequent cyclization reaction, it is preferred to use sodium azide in a molar amount of 2 to 6 times to that of the imidoyl chloride and in general, the reaction is preferably performed at a temperature of not higher than room temperature in N,N-dimethylformamide. In the case of using hydrogen azide, the reaction is preferably performed in a molar amount of 2 to 3 times to that of the imidoyl chloride and the reaction is performed, in general, in benzene at room temperature or under reflux. The compound of Formula (II) can be produced by the method as undermentioned, according to the method described in Tetrahedron Letters, 1595, (1973).

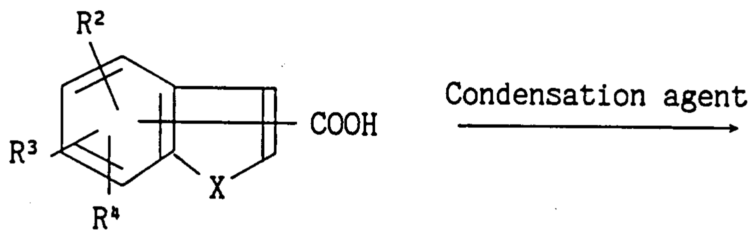


[IV]

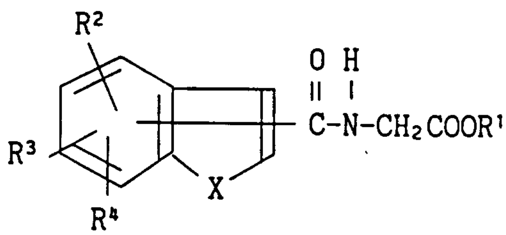

[II]

The compound having Formula (II) can be prepared by condensation of the carboxylic acid (Formula IV) with alkyl glycine ester hydrochloride salt in the presence of triethylamine in a molar amount of 2 times that of the carboxylic acid and a condensation agent such as diethyl phosphorocyanidate (DEPC) or diphenylphosphoryl azide (DPPA) in a molar amount of 1 to 1.1 times that of the carboxylic acid. Furthermore, also conventional condensation agents, such as dicyclohexylcarbodiimide (DCC) may be utilized in this reaction. Some of the carboxylic acid derivatives shown in Formula (IV) as the starting material are commercially available and the others can be prepared by known chemical methods. The synthesis method of a benzothiophene derivative is described in the Journal of Heterocyclic Chemistry vol.12, 889 (1975) and that of a benzofurane derivative in the Journal of Organic Chemistry vol.42, 4265 (1977) separately.

The reaction scheme 2 shows that the compounds of Formula (I) in which $R^1$ is a hydrogen atom may be prepared by hydrolysis of the carboxylic acid ester obtained in the reaction scheme 1. The hydrolysis can be performed in the presence of a base, such as sodium hydroxide or potassium hydroxide, or an acid, such as hydrochloric acid, sulfuric acid, acetic acid or trifluoroacetic acid.

The compounds of Formula (I) prepared according to the foregoing method are separated and purified by chemical operations commonly employed such as extraction, recrystallization and/or column chromatography and the products thus separated and purified are used as essential components for the aldose reductase inhibitors of the present invention.

The present invention will hereinafter be described in more detail with reference to the following non-limitative working Examples and the effects practically achieved by the present invention will also be discussed in detail with reference to Test Examples.

Example 1

(1-1) Preparation of methyl[5-(2-benzothienyl)tetrazol-1-yl]acetate;

To a solution of 200 mg (0.80 mM) of N-(2-benzothenoyl) glycine methyl ester in 2 ml of anhydrous methylene chloride, there was slowly added 190 mg (0.91 mM) of phosphorus pentachloride at room temperature with stirring, the resulting mixture was stirred for additional 30 minutes and the reaction solution was concentrated at 40°C under reduced pressure. The resulting residue was dissolved in 2 ml of N,N-dimethylformamide. This solution was then dropwise added to an ice cooled suspension of 140 mg (2.15 mM) of sodium azide in 2 ml of N-N-dimethylformamide with stirring. After the completion of addition, the mixture was stirred at room temperature. The mixture was then poured into ice-water and extracted with ethyl acetate. The organic phase was washed with water, dried over anhydrous sodium sulfate and then concentrated under reduced pressure. The resultant residue was subjected to silica gel column chromatography (eluent: ethyl acetate/benzene = 1/9) for separation and purification to thus give 99 mg (yield 44.8%) of methyl[5-(2-benzothienyl)tetrazol-1-yl]acatate.

| | |
|---|---|
| M.P. | : 130-131°C |
| N.M.R.($CDCl_3$) $\delta$ | : 3.84(s,3H), 5.43(s,2H), 7.47-7.53(m,2H), 7.84(s,1H), 7.90-7.94(m,2H) |
| I.R.$\nu_{KBr}$ $cm^{-1}$ | : 3440, 1750, 1440, 1400, 1220, 1120, 1000 |
| Mass | : m/z 274[$M^+$] |

The following compounds were prepared in the same manner as Example (1-1)

(1-2) Methyl[5-(3-benzothienyl)tetrazol-1-yl]acetate yield = 42.4%

| | |
|---|---|
| Starting material | : N-(3-benzothenoyl)glycine methyl ester |
| M.P. | : 146.5-147°C |
| N.M.R.($CDCl_3$) $\delta$ | : 3.82(s,3H), 5.43(s,2H), 7.46-7.55(m,2H), 7.84(s,1H), 7.92-7.98(m,1H), 8.07-8.13-(m,1H) |
| I.R.$\nu_{KBr}$ $cm^{-1}$ | : 3450, 1760, 1580, 1430, 1360, 1240, 1220 |
| Mass | : m/z 274[$M^+$] |

(1-3) Methyl[5-(4-benzothienyl)tetrazol-1-yl]acetate yield = 40%

| | |
|---|---|
| Starting material | : 4-(methoxycarbonylmethylaminocarbonyl) benzothiophene |
| N.M.R.($CDCl_3$) $\delta$ | : 3.69(s,3H), 5.19(s,2H), 7.37-7.54(m,4H), 7.95(dd,1H,J = 8.10,1.20Hz) |
| I.R.$\nu_{NaCl}$ $cm^{-1}$ | : 3100, 2950, 1750, 1430, 1260, 1220, 1100, 800 |
| Mass | : m/z 274[$M^+$] |

(1-4) Methyl[5-(6-benzothienyl)tetrazol-1-yl]acetate yield = 36%

| | |
|---|---|
| Starting material | : 6-(methoxycarbonylmethylaminocarbonyl) benzothiophene |
| N.M.R.($CDCl_3$) $\delta$ | : 3.82(s,3H), 5.25(s,2H), 7.45(dd,1H,J = 5.20,0.80Hz), 7.59(dd,1H,J = 8.10,1.60Hz), 7.66(d,1H,J = 5.20Hz), 7.99(d,1H,J = 8.10Hz), 8.22(t,1H,J = 0.80Hz) |
| I.R.$\nu_{NaCl}$ $cm^{-1}$ | : 3100, 2950, 1750, 1530, 1440, 1260, 1220 |
| Mass | : m/z 274[$M^+$] |

(1-5) Methyl[5-(3-methyl-2-benzothienyl)tetrazol-1-yl]acetate yield = 41%

| | |
|---|---|
| Starting material | : N-(4-methyl-2-benzothenoyl)glycine methyl ester |
| N.M.R.($CDCl_3$) $\delta$ | : 2.58(s,3H), 3.79(s,3H), 5.30(s,2H), 7.45-7.52(m,2H), 7.85-7.90(m,2H) |
| I.R.$\nu_{NaCl}$ $cm^{-1}$ | : 3450, 1760, 1430, 1240, 1220, 990 |
| Mass | : m/z 288[$M^+$] |

(1-6) Methyl[5-(3-chloro-2-benzothienyl)tetrazol-1-yl]acetate yield = 31%

| | |
|---|---|
| Starting material | : N-(3-chloro-2-benzothenoyl)glycine methyl ester |
| N.M.R.($CDCl_3$) $\delta$ | : 3.68(s,3H), 5.27(s,2H), 7.48-7.55(m,2H), 7.80-7.93(m,2H) |
| I.R.$\nu_{NaCl}$ $cm^{-1}$ | : 3230, 3060, 1760, 1650, 1600, 1550, 1440, 1340, 1280, 1220, 1000 |
| Mass | : m/z 308[$M^+$] |

(1-7) Methyl[5-(4-methyl-2-benzothienyl)tetrazol-1-yl]acetate yield = 71.2%

Starting material : N-(4-methyl-2-benzothenoyl)glycine methyl ester
N.M.R.($CDCl_3$) δ : 2.55(s,3H), 3.74(s,3H), 5.34(s,2H), 7.15(d,1H,J = 7.32Hz), 7.28(t,1H,J = 7.33Hz), 7.63(d,1H,J = 8.06Hz), 7.86(d,1H,J = 0.73Hz)
I.R.$\nu_{KBr}$ $cm^{-1}$ : 2950, 1760, 1580, 1440, 1220, 770
Mass : m/z 288[$M^+$]

(1-8) Methyl[5-(6-methoxy-2-benzothienyl)tetrazol-1-yl]acetate yield = 65.2%

Starting material : N-(6-methyl-2-benzothenoyl)glycine methyl ester
M.P. : 145-146 ° C
N.M.R.($CDCl_3$) δ : 2.52(s,3H), 3.84(s,3H), 5.41(s,2H), 7.28(dd,1H,J = 8.30,0.73Hz), 7.71-(d,1H,J = 0.73Hz), 7.78(s,1H), 7.80(d,1H,J = 8.30Hz)
I.R.$\nu_{KBr}$ $cm^{-1}$ : 2950, 1760, 1630, 1440, 1220, 1120
Mass : m/z 288[$M^+$]

(1-9) Methyl[5-(4-methoxy-2-benzothienyl)tetrazol-1-yl]acetate yield = 67.9%

Starting material : N-(4-methoxy-2-benzothenoyl)glycine methyl ester
M.P. : 84-85 ° C
N.M.R.($CDCl_3$) δ : 3.84(s,3H), 3.98(s,3H), 5.41(s,2H), 6.81(dd,1H,J = 7.33,0.98Hz), 7.38-7.49(m,2H), 7.93(d,1H,J = 0.49Hz)
I.R.$\nu_{KBr}$ $cm^{-1}$ : 3000, 2950, 1750, 1580, 1410, 1260, 1030, 770
Mass : m/z 304[$M^+$]

(1-10)Methyl[5-(5-methoxy-2-benzothienyl)tetrazol-1-yl]acetate yield = 73.4%

Starting material : N-(5-methoxy-2-benzothenoyl)glycine methyl ester
M.P. : 132-133 ° C
N.M.R.($CDCl_3$) δ : 3.85(s,3H), 3.90(s,3H), 5.36(s,2H), 7.13(dd,1H,J = 8.86,2.42Hz), 7.32-(d,1H,J = 2.42Hz), 7.77(d,1H,J = 8.86Hz), 7.78(s,1H)
I.R.$\nu_{KBr}$ $cm^{-1}$ : 2950, 1750, 1600, 1550, 1430, 1240, 1220, 1000, 820, 680
Mass : m/z 304[$M^+$]

(1-11)Methyl[5-(6-methoxy-2-benzothienyl)tetrazol-1-yl]acetate yield = 67.9%

Starting material : N-(6-methoxy-2-benzothenoyl)glycine methyl ester
M.P. : 144-145 ° C
N.M.R.($CDCl_3$) δ : 3.85(s,3H), 3.91(s,3H), 5.40(s,2H), 7.08(dd,1H,J = 8.79,2.19Hz), 7.34-(d,1H,J = 2.20Hz), 7.72(s,1H), 7.78(d,1H,J = 8.79Hz)
I.R.$\nu_{KBr}$ $cm^{-1}$ : 3000, 2950, 1750, 1580, 1410, 1280, 1110, 1060, 870, 800
Mass : m/z 304[$M^+$]

(1-12)Methyl[5-(6-butoxy-2-benzothienyl)tetrazol-1-yl]acetate yield = 59%

Starting material : N-(6-butoxy-2-benzothenoyl)glycine methyl ester
M.P. : 134-135 ° C
N.M.R.($CDCl_3$) δ : 1.00(t,3H,J = 7.32Hz), 1.46-1.57(m,2H), 1.78-1.88(m,2H), 3.85(s,3H), 4.06-(t,2H,J = 6.47Hz), 5.40(s,2H), 7.07(dd,1H,J = 8.79,2.20Hz), 7.33(d,1H,J = 2.20Hz), 7.71(s,1H), 7.77(d,1H,J = 8.79Hz)
I.R.$\nu_{KBr}$ $cm^{-1}$ : 2950, 1750, 1580, 1440, 1380, 1260, 1230, 1110, 1070, 980, 840
Mass : m/z 346[$M^+$]

(1-13)Methyl[5-(6-isopropyl-2-benzothienyl)tetrazol-1-yl] acetate yield = 47.9%

Starting material : N-(6-isopropyl-2-benzothenoyl)glycine methyl ester
M.P. : 109-110 ° C
N.M.R.($CDCl_3$) δ : 1.33(d,6H,J = 7.08Hz), 3.07(quint,1H,J = 6.83Hz), 3.83(s,3H), 5.41(s,2H), 7.35-(dd,1H,J = 8.30,1.46Hz), 7.75-7.79(m,2H), 7.83(d,1H,J = 8.30Hz)

I.R.$\nu_{KBr}$ $cm^{-1}$ : 2950, 1740, 1590, 1430, 1380, 1240, 850, 800
Mass : m/z 316[$M^+$]

(1-14)Methyl[5-(5,6,7-trimethoxy-2-benzothienyl)tetrazol-1-yl] acetate yield = 47.3%

Starting material : N-(5,6,7-trimethoxy-2-benzothenoyl) glycine methyl ester
M.P. : 142-143 ° C
N.M.R.($CDCl_3$) δ : 3.85(s,3H), 3.95(s,3H), 3.96(s,3H), 4.12(s,3H), 5.41(s,2H), 7.10(s,1H), 7.75(s,1H)
I.R.$\nu_{KBr}$ $cm^{-1}$ : 3000, 2950, 1760, 1590, 1480, 1390, 1220, 1100, 1000
Mass : m/z 364[$M^+$]

(1-15)Methyl[5-(2-benzofuryl)tetrazol-1-yl]acetate yield = 34%

Starting material : N-(2-benzofuroyl)glycine methyl ester
M.P. : 114.5-115 ° C
N.M.R.($CDCl_3$) δ : 3.86(s,3H), 5.62(s,2H), 7.33-7.76(m,5H)
I.R.$\nu_{KBr}$ $cm^{-1}$ : 3410, 1760, 1620, 1440, 1260, 1220
Mass : m/z 258[$M^+$]

(1-16)Methyl[5-(3-methyl-2-benzofuryl)tetrazol-1-yl]acetate yield = 61.8%

Starting material : N-(3-methyl-2-benzofuroyl)glycine methyl ester
M.P. : 144-145 ° C
N.M.R.($CDCl_3$) δ : 2.76(s,3H), 3.78(s,3H), 5.62(s,2H), 7.34-7.49(m,3H), 7.68(d,1H,J = 7.57Hz)
I.R.$\nu_{KBr}$ $cm^{-1}$ : 3020, 2950, 1760, 1620, 1520, 1440, 1420, 1280, 1150, 1000, 750
Mass : m/z 260[$M^+$]

(1-17)Methyl[5-(6-methoxy-3-methyl-2-benzofuryl)tetrazol-1-yl] acetate yield = 52%

Starting material : N-(6-methoxy-3-methyl-2-benzofuroyl) glycine methyl ester
M.P. : 128-129 ° C
N.M.R.($CDCl_3$) δ : 2.72(s,3H), 3.78(s,3H), 3.89(s,3H), 5.59(s,2H), 6.96(d,1H,J = 1.46Hz), 6.98-(dd,1H,J = 8.55,2.20Hz), 7.54(d,1H,J = 8.55Hz)
I.R.$\nu_{KBr}$ $cm^{-1}$ : 3000, 2950, 1750, 1620, 1500, 1440, 1280
Mass : m/z 290[$M^+$]

Example 2

(2-1) [5-(2-Benzothienyl)tetrazol-1-yl]acetic acid

10 ml of 2N sodium hydroxide aqueous solution was added to 5 ml of ethanol solution containing 200 mg (0.73 mM) of methyl [5-(2-benzothienyl)tetrazol-1-yl]acetate. The mixture was refluxed for 1 hr, and cooled. The solution was concentrated under reduced pressure. The resultant residue was dissolved in water. The aqueous solution was acidified with hydrochloric acid. The resulting precipitate was collected by filtration, washed with water and recrystallized from 30% ethanol-water mixture to give 144 mg (yield 76%) of [5-(2-benzothienyl)tetrazol-1-yl]acetic acid.

M.P. : 225-226 ° C (decomposition)
N.M.R.(DMSO-$d_6$) δ : 3.86(bs,1H), 5.40(s,2H), 7.46-7.50(m,2H), 7.85(s,1H), 7.91-7.94(m,2H)
I.R.$\nu_{KBr}$ $cm^{-1}$ : 3420, 2950, 1730, 1580, 1240
Mass : m/z 260[$M^+$]

The following compounds were prepared in the same manner as Example (2-1)

(2-2) [5-(3-Benzothienyl)tetrazol-1-yl]acetic acid yield = 72%

Starting material : methyl[5-(3-benzothienyl)tetrazol-1-yl]acetate
M.P. : 179-180 ° C (decomposition)
N.M.R.(DMSO-$d_6$) δ : 3.96(bs,1H), 5.27(s,2H), 7.48-7.52(m,2H), 7.96-8.02(m,2H), 8.15-8.19(m,1H)
I.R.$\nu_{KBr}$ $cm^{-1}$ : 3450, 2900, 1730, 1570, 1430, 1410, 1230, 1210

Mass : m/z 260[$M^+$]

(2-3) [5-(4-Benzothienyl)tetrazol-1-yl]acetic acid yield = 76%

Starting material : methyl[5-(4-benzothienyl)tetrazol-1-yl]acetate
M.P. : 113-115 ° C (decomposition)
N.M.R.(DMSO-$d_6$) $\delta$ : 3.54(bs,1H), 5.27(s,2H), 7.43-7.63(m,3H), 8.01-8.06(m,2H)
I.R.$\nu_{KBr}$ $cm^{-1}$ : 3510, 3380, 1720, 1620, 1440, 1350, 1270, 1240, 1120
Mass : m/z 260[$M^+$]

(2-4) [5-(6-Benzothienyl)tetrazol-1-yl]acetic acid yield = 72%

Starting material : methyl[5-(6-benzothienyl)tetrazol-1-yl]acetate
M.P. : 179-180 ° C (decomposition)
N.M.R.(DMSO-$d_6$) $\delta$ : 4.39(bs,1H), 5.23(s,2H), 7.45(d,1H,J = 8.20Hz), 7.62-7.69(m,2H), 8.00-(d,1H,J = 8.50Hz), 8.26(s,1H)
I.R.$\nu_{KBr}$ $cm^{-1}$ : 3500, 3000, 2600, 1730, 1460, 1250, 1230
Mass : m/z 260[$M^+$]

(2-5) [5-(3-Methyl-2-benzothienyl)tetrazol-1-yl]acetic acid yield = 76%

Starting material : methyl[5-(3-methyl-2-benzothienyl) tetrazol-1-yl]acetate
M.P. : 153-154 ° C (decomposition)
N.M.R.(DMSO-$d_6$) $\delta$ : 2.56(s,3H), 4.11(bs,1H), 5.24(s,2H), 7.48-7.51(m,2H), 7.84-7.90(m,2H)
I.R.$\nu_{KBr}$ $cm^{-1}$ : 3450, 1740, 1590, 1430, 1220
Mass : m/z 274[$M^+$]

(2-6) [5-(3-Chloro-2-benzothienyl)tetrazol-1-yl]acetic acid yield = 61%

Starting material : methyl[5-(3-chloro-2-benzothienyl) tetrazol-1-yl]acetate
M.P. : 144-145 ° C (decomposition)
N.M.R.(DMSO-$d_6$) $\delta$ : 5.30(s,2H), 5.50(bs,1H), 7.55-7.62(m,2H), 7.89-8.01(m,2H)
I.R.$\nu_{KBr}$ $cm^{-1}$ : 3440, 2920, 1740, 1590, 1440, 1220
Mass : m/z 294[$M^+$]

(2-7) [5-(4-Methyl-2-benzothienyl)tetrazol-1-yl]acetic acid yield = 72%

Starting material : methyl[5-(4-methyl-2-benzothienyl) tetrazol-1-yl]acetate
M.P. : 224-225 ° C (decomposition)
N.M.R.($CDCl_3$ + DMSO-$d_6$) $\delta$ : 2.66(s,3H), 5.42(s,2H), 7.25(d,1H,J = 7.32Hz), 7.39-(dd,1H,J = 8.06,7.32Hz), 7.75(d,1H,J = 8.06Hz), 7.97(s,1H)
I.R.$\nu_{KBr}$ $cm^{-1}$ : 2950, 1740, 1580, 1400, 1220, 1120, 820
Mass : m/z 274[$M^+$]

(2-8) [5-(6-Methyl-2-benzothienyl)tetrazol-1-yl]acetic acid yield = 86.7%

Starting material : methyl[5-(6-methyl-2-benzothienyl) tetrazol-1-yl]acetate
M.P. : 196-197 ° C (decomposition)
N.M.R.($CDCl_3$ + DMSO-$d_6$) $\delta$ : 2.52(s,3H), 5.44(s,2H), 7.28(d,1H,J = 8.30Hz), 7.73(s,1H), 7.81-(d,1H,J = 8.06Hz), 7.82(s,1H)
I.R.$\nu_{KBr}$ $cm^{-1}$ : 2970, 1730, 1580, 1440, 1260, 1120
Mass : m/z 274[$M^+$]

(2-9) [5-(4-Methoxy-2-benzothienyl)tetrazol-1-yl]acetic acid yield = 72.1%

Starting material : methyl[5-(4-methoxy-2-benzothienyl) tetrazol-1-yl]acetate
M.P. : 189-190 ° C (decomposition)
N.M.R.($CDCl_3$ + DMSO-$d_6$) $\delta$ : 3.99(s,3H), 3.63(bs,1H), 5.39(s,2H), 6.83(d,1H,J = 7.57Hz), 7.43-

| | |
|---|---|
| | (d,1H,J = 7.57Hz), 7.47-7.50(m,1H), 7.95(d,1H,J = 0.49Hz) |
| I.R.$\nu_{KBr}$ $cm^{-1}$ | : 3000, 1740, 1580, 1570, 1420, 1260, 1220, 1050 |
| Mass | : m/z 290[$M^+$] |

(2-10)[5-(5-Methoxy-2-benzothienyl)tetrazol-1-yl]acetic acid yield = 86.3%

| | |
|---|---|
| Starting material | : methyl[5-(5-methoxy-2-benzothienyl) tetrazol-1-yl]acetate |
| M.P. | : 213-214 ° C (decomposition) |
| N.M.R.($CDCl_3$ + DMSO-$d_6$) $\delta$ | : 3.85(s,3H), 5.71(s,2H), 7.17(dd,1H,J = 8.80,2.56Hz), 7.54-(d,1H,J = 2.56Hz), 8.00(d,1H,J = 8.80Hz), 8.02(s,1H) |
| I.R.$\nu_{KBr}$ $cm^{-1}$ | : 2930, 1740, 1580, 1400, 1300, 1235, 1160, 1025 |
| Mass | : m/z 290[$M^+$] |

(2-11)[5-(6-Methoxy-2-benzothienyl)tetrazol-1-yl]acetic acid yield = 86.3%

| | |
|---|---|
| Starting material | : methyl[5-(6-methoxy-2-benzothienyl) tetrazol-1-yl]acetate |
| M.P. | : 207-208 ° C (decomposition) |
| N.M.R.($CDCl_3$ + DMSO-$d_6$) $\delta$ | : 3.92(s,3H), 5.41(s,2H), 7.07(dd,1H,J = 8.79,2.44Hz), 7.38-(d,1H,J = 2.20Hz), 7.76(s,1H), 7.80(d,1H,J = 8.79Hz) |
| I.R.$\nu_{KBr}$ $cm^{-1}$ | : 3000, 1730, 1580, 1270, 1230, 1100, 1010, 840, 670 |
| Mass | : m/z 290[$M^+$] |

(2-12)[5-(6-Butoxy-2-benzothienyl)tetrazol-1-yl]acetic acid yield = 74.4%

| | |
|---|---|
| Starting material | : methyl[5-(6-butoxy-2-benzothienyl) tetrazol-1-yl]acetate |
| M.P. | : more than 300 ° C |
| N.M.R.(DMSO-$d_6$) $\delta$ | : 0.96(t,3H,J = 7.33Hz), 1.43-1.51(m,2H), 1.70-1.77(m,2H), 3.48(bs,1H), 4.07-(t,2H,J = 6.59Hz), 5.23(s,2H), 7.04(dd,1H,J = 8.79,2.44Hz), 7.65(d,1H,J = 2.19Hz), 7.83(d,1H,J = 8.79Hz) |
| I.R.$\nu_{KBr}$ $cm^{-1}$ | : 2950, 1730, 1620, 1580, 1550, 1460, 1390, 1270, 1220, 1060 |
| Mass | : m/z 332[$M^+$] |

(2-13)[5-(6-Isopropyl-2-benzothienyl)tetrazol-1-yl]acetic acid yield = 96%

| | |
|---|---|
| Starting material | : methyl[5-(6-isopropyl-2-benzothienyl)tetrazol-1-yl]acetate |
| M.P. | : 193-194 ° C (decomposition) |
| N.M.R.($CDCl_3$ + DMSO-$d_6$) $\delta$ | : 1.33(d,6H,J = 7.08Hz), 5.07(quint,1H,J = 6.83Hz), 4.68(bs,1H), 5.40-(s,2H), 7.36(dd,1H,J = 8.30,1.46Hz), 7.76-7.80(m,2H), 7.84-(d,1H,J = 8.30Hz) |
| I.R.$\nu_{KBr}$ $cm^{-1}$ | : 2950, 1740, 1590, 1250, 850, 810 |
| Mass | : m/z 302[$M^+$] |

(2-14)[5-(5,6,7-Trimethoxy-2-benzothienyl)tetrazol-1-yl]acetic acid yield = 85.1%

| | |
|---|---|
| Starting material | : methyl[5-(5,6,7-trimethoxy-2-benzothienyl)tetrazol-1-yl]acetate |
| M.P. | : 177-178 ° C (decomposition) |
| N.M.R.($CDCl_3$ + DMSO-$d_6$) $\delta$ | : 3.95(s,6H), 4.11(s,3H), 5.41(s,2H), 7.15(s,1H) |
| I.R.$\nu_{KBr}$ $cm^{-1}$ | : 2950, 1740, 1580, 1400, 1240, 1120, 1020, 860 |
| Mass | : m/z 350[$M^+$] |

(2-15)[5-(2-Benzofuryl)tetrazol-1-yl]acetic acid yield = 55%

| | |
|---|---|
| Starting material | : methyl[5-(2-benzofuryl)tetrazol-1-yl] acetate |
| M.P. | : 182-183 ° C (decomposition) |
| N.M.R.(DMSO-$d_6$) $\delta$ | : 4.17(bs,1H), 5.59(s,2H), 7.34-7.77(m,5H) |
| I.R.$\nu_{KBr}$ $cm^{-1}$ | : 3470, 1710, 1620, 1450, 1340, 1260, 1240 |
| Mass | : m/z 244[$M^+$] |

(2-16)[5-(3-Methyl-2-Benzofuryl)tetrazol-1-yl]acetic acid yield = 73.8%

| | |
|---|---|
| Starting material | : methyl[5-(3-methyl-2-benzofuryl) tetrazol-1-yl]acetate |
| M.P. | : 194-195 °C (decomposition) |
| N.M.R.($CDCl_3$ + DMSO-$d_6$) $\delta$ | : 2.74(s,3H), 5.58(s,2H), 7.33-7.39(m,1H), 7.45(td,1H,J = 7.03,1.22Hz), 7.50-7.54(m,1H), 7.69(d,1H,J = 7.57Hz) |
| I.R.$\nu_{KBr}$ $cm^{-1}$ | : 3020, 2950, 1750, 1620, 1520, 1440, 1280, 1150, 1000, 780 |
| Mass | : m/z 246[$M^+$] |

(2-17)[5-(6-Methoxy-3-methyl-2-benzofuryl)tetrazol-1-yl]acetic acid yield = 83.9%

| | |
|---|---|
| Starting material | : methyl[5-(6-methoxy-3-methyl-2-benzofuryl)tetrazol-1-yl]acetate |
| M.P. | : 213-214 °C (decomposition) |
| N.M.R.($CDCl_3$ + DMSO-$d_6$) $\delta$ | : 2.69(s,3H), 3.89(s,3H), 5.56(s,2H), 6.86(dd,1H, J = 8.54,2.20Hz), 7.02-(d,1H,J = 1.96Hz), 7.54(d,1H,J = 8.54Hz) |
| I.R.$\nu_{KBr}$ $cm^{-1}$ | : 2950, 1740, 1620, 1500, 1440, 1280, 1160, 800 |
| Mass | : m/z 276[$M^+$] |

As has been explained above in detail, the aldose reductase inhibitor of the present invention shows excellent aldose reductase inhibitory effect and has a low toxicity. Therefore, it can be used as a medicine for preventing and/or treating mammalian inclusive of man suffering from diabetic complications such as neural disorders, nephrosis, cataract and retinopathy with safety.

The effects and toxicity of the aldose reductase inhibitor according to the present invention will be shown in more detail below with reference to the following Test Examples.

Test Example 1: Test for Examining Aldose Reductase Inhibitory Effect

(i) Methodology

Six-week-old male SD rats were anesthetized with ether and killed. Then their crystalline lenses were immediately removed and stored at -80 °C . The lenses were homogenized in 3 volumes of 135 mM sodium potassium phosphate buffer (pH 7.0) and centrifuged at 30,000 rpm for 30 minutes. The resulting supernatant was dialyzed overnight against 0.05 M sodium chloride solution to obtain an aldose reductase solution. All operations were conducted at 4 °C and the enzyme solution was stored at -80 °C .

The activity of aldose reductase was determined according to a partially modified method of J.H. Kinoshita et al. (J. Biol. Chem., 1965, 240, p. 877). More specifically, 0.1 ml of DL-glyceraldehyde (final concentration: 10 mM) was added to 0.9 ml of 100 mM sodium potassium phosphate buffer (pH 5.2) which contained lithium sulfate (final concentration: 400 mM), reduced nicotinamide adenine dinucleotide phosphate (final concentration: 0.15 mM), the enzyme solution, and the compound to be evaluated (final concentration: $10^{-6}$ M, $10^{-7}$ M or $10^{-8}$ M), and then the reaction was conducted at 30 °C for 5 minutes. During the reaction, the change in the absorbance at 340 nm with time was monitored.

The maximum reducing rate of the absorbance (U) during the reaction was determined. By subtracting, from this value, the maximum reducing rate ($U_0$) at 340 nm of the reaction solution before the addition of the substrate (DL-glyceraldehyde), the reaction rate ($V = U - U_0$) was calculated as a true reaction rate in the presence of the compound to be tested. The same procedure was repeated except for the absence of the compound to be tested. A true reaction rate ($V_0$) in case the enzyme was not inhibited was calculated ($V_0 = U^1 - U_0{}^1$). The aldose reductase inhibitory activity of the test compounds was determined according to the following formula:

$$\text{Rate of Inhibition (\%)} = (V_0 - V)/V_0 \times 100$$

The concentration of inhibitor giving 50% inhibition of enzyme activity ($IC_{50}$) was estimated from the least-square regression line of the log dose-response curve.

For comparison, the same tests were conducted using a known aldose reductase inhibitor: ONO-2235 [-(E)-3-carboxymethyl-5-[(2E)-methyl-3-phenylpropenylidene]rhodan].

(ii) Results

The results thus obtained are summarized in the following Table 1.

As seen from Table I, the compounds of the present invention tested show aldose reductase inhibitory effect identical to or superior to those attained by the known inhibitor ONO-2235.

Table 1

| Compound tested (Example No.) | $IC_{50}$ (x $10^{-8}$) M | Compound tested (Example No.) | $IC_{50}$ (x $10^{-8}$) M |
|---|---|---|---|
| 2-1 | 2.4 | 2-10 | 2.8 |
| 2-2 | 1.9 | 2-11 | 2.1 |
| 2-3 | 2.6 | 2-12 | 2.4 |
| 2-4 | 3.4 | 2-13 | 1.8 |
| 2-5 | 3.2 | 2-14 | 6.9 |
| 2-6 | 3.5 | 2-15 | 4.6 |
| 2-7 | 2.2 | 2-16 | 6.8 |
| 2-8 | 1.8 | 2-17 | 16 |
| 2-9 | 2.0 | | |
| ONO-2235 | 3.1 | | |

## Claims

1. A tetrazoleacetic acid derivative represented by the following general Formula (I):

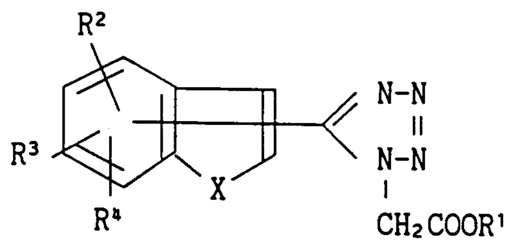

(I)

[in Formula (I), $R^1$ represents a hydrogen atom or a lower alkyl group; $R^2$, $R^3$ and $R^4$ are the same or different from each other and are selected from the group consisting of a hydrogen atom, a lower alkyl group, a halogen atom and a lower alkoxy group; X is an oxygen atom or a sulfur atom; and the tetrazole group substituent may be present arbitrarily at the 2-,3-,4-,5-, 6- and 7-position of the benzofurane or benzothiophene ring] or a salt thereof.

2. The tetrazoleacetic acid derivative of claim 1 wherein, in general Formula (I), the lower alkyl group represented by $R^1$ is a member selected from the group consisting of methyl, ethyl, propyl, isopropyl, butyl, isobutyl and t-butyl group; the lower alkyl group represented by $R^2$, $R^3$ or $R^4$ is a member selected from the group consisting of methyl, ethyl, propyl, isopropyl, butyl, iso-butyl and t-butyl group; the halogen atom represented by $R^2$, $R^3$ or $R^4$ is a member selected from the group consisting of fluorine, chlorine or bromine; the lower alkoxy group represented by $R^2$, $R^3$ or $R^4$ is a member selected from the group consisting of methoxy, ethoxy, propoxy, isopropoxy, butoxy, isobutoxy and t-butoxy group; and the substituents $R^2$, $R^3$ and $R^4$ may be present at any position on the benzofurane or the benzothiophene ring.

3. The tetrazoleacetic acid derivative of claim 1 wherein the salt of the compound represented by Formula (I) wherein $R^1$ is a hydrogen atom is a member selected from the group consisting of alkali metal salts, alkaline earth metal salts, ammonium salts, organic amine salts, and salts with basic amino acids.

4. The tetrazoleacetic acid derivative of claim 3 wherein the alkali metal salt is a sodium salt or a potassium salt; the alkaline earth metal salt is a calcium salt or a magnesium salt; the organic amine salt is a triethylamine salt, a pyridine salt or an ethanolamine salt; and the basic amino acid salt is an arginine salt.

**5.** The tetrazoleacetic acid derivative of claim 1 wherein, in the general Formula (I), $R^1$ is a hydrogen atom and X is an oxygen or sulfur atom.

**6.** An aldose reductase inhibitor comprising a tetrazoleacetic acid derivative represented by the following general Formula (I):

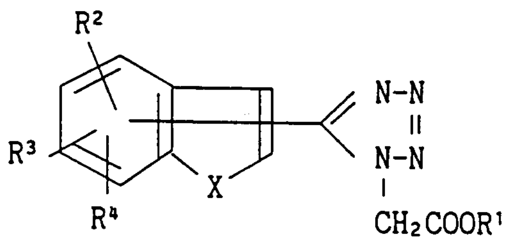

(I)

[in Formula (I), $R^1$ represents a hydrogen atom or a lower alkyl group; $R^2$, $R^3$ and $R^4$ are the same or different from each other and are selected from the group consisting of a hydrogen atom, a lower alkyl group, a halogen atom and a lower alkoxy group; X is an oxygen atom or a sulfur atom; and the tetrazole group substituent may be present arbitrarily at the 2-,3-,4-,5-,6-, and 7-position of the benzofurane or benzothiophene ring] or a salt thereof and a pharmaceutically acceptable carrier.

**7.** The aldose reductase inhibitor of claim 6 wherein, in the general Formula (I); the lower alkyl group represented by $R^1$ is a member selected from the group consisting of methyl, ethyl, propyl, isopropyl, butyl, isobutyl and t-butyl group; the lower alkyl group represented by $R^2$, $R^3$ or $R^4$ is a member selected from the group consisting of methyl, ethyl, propyl, isopropyl, butyl, isobutyl and t-butyl group; the halogen atom represented by $R^2$, $R^3$ or $R^4$ is a member selected from the group consisting of fluorine, chlorine or bromine; the lower alkoxy group represented by $R^2$, $R^3$ or $R^4$ is a member selected from the group consisting of methoxy, ethoxy, propoxy, isopropoxy, butoxy, isobutoxy and t-butoxy group; and the substituents $R^2$, $R^3$ and $R^4$ may be present at any position on the benzofurane or benzothiophene ring.

**8.** The aldose reductase inhibitor of claim 6 wherein the salt of the compound represented by Formula (I) wherein $R^1$ is a hydrogen atom, is a member selected from the group consisting of alkali metal salts, alkaline earth metals, ammonium salts, organic amine salts and salts with basic amino acids.

**9.** The aldose reductase inhibitor of claim 8 wherein the alkali metal salt is a sodium salt or potassium salt; the alkaline earth metal salt is a calcium salt or a magnesium salt; the organic amine salt is a triethylamine salt, a pyridine salt or an ethanolamine salt; and the basic amino acid salt is an arginine salt.

**10.** The aldose reductase inhibitor of claim 6 wherein, in general Formula (I), $R^1$ is a hydrogen atom and X is an oxygen or a sulfur atom.

**11.** Use of the tetrazoleacetic acid derivatives according to any of claims 1 to 5 for the preparation of a pharmaceutical composition for alleviating or reducing diabetic complications.

**12.** The use of claim 11 wherein the compound of Formula (I) in the pharmaceutical composition is to be administered at one time or over several times, in an amount ranging from 1 to 1000 mg per day for adults, orally, subcutaneously, intravenously or locally.

**13.** The use of claim 11 wherein the compound of Formula (I) in the pharmaceutical composition is to be administered in the form of tablets, powders, fine particles, granules, capsules, pills, liquid preparations, solutions or suspensions for injection or eye drops.

European Patent Office

# EUROPEAN SEARCH REPORT

Application Number

EP 92 10 5134

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 388 967 (WAKAMOTO PHARMACEUTICAL CO., LTD.)<br>* page 3, line 1 - page 9, line 21; claims *<br>--- | 1-13 | C07D405/04<br>C07D409/04<br>A61K31/41 |
| P,D,Y | EP-A-0 421 365 (WAKAMOTO PHARMACEUTICAL CO., LTD.)<br>* page 3, line 1 - page 7, line 2; claims *<br>----- | 1-13 | |
| | | | **TECHNICAL FIELDS SEARCHED (Int. Cl.5)** |
| | | | C07D |

The present search report has been drawn up for all claims

| Place of search | Date of completion of the search | Examiner |
|---|---|---|
| THE HAGUE | 09 JUNE 1992 | PAISDOR B. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document